# EUROPEAN PATENT APPLICATION

(11) **EP 1 393 728 A1**
(43) Date of publication of application: **03.03.2004**
(21) Application number: 02078598.6
(22) Date of filing: 30.08.2002
(51) Int. Cl.: A61K 31/4418, C07D 211/52, A61P 31/18

(54) **Inverse agonists acting at virus-encoded G protein-coupled receptors**

(71) Applicant: VRIJE UNIVERSITEIT, 1081 HV Amsterdam (NL)
(72) Inventor: Leurs, Rob, 1075 CB Amsterdam (NL); Menge, Wiro Michael Petrus Bernardus, 6821 HM Arnhem (NL); Smit, Martine Joyce, 1066 HR Amsterdam (NL); Casarosa, Paola, 1092 GG Amsterdam (NL)
(74) Representative: Prins, Adrianus Willem

(57) **Abstract**

The present invention relates to a group of non-peptidergic compounds which compounds are inverse agonists acting on the Human cytomegalovirus (HCMV) encoded US28 receptor. In addition, the invention relates to the use of these compounds in the manufacture of a medicament having inverse agonist activity on HCMV encoded US28 receptor. Further, the invention relates to the use of these compounds in the manufacture of a medicament for inhibiting viral infections, and particularly in the treatment of HIV infections, such as in AIDS.

## Description

The present invention relates to inverse agonists acting at G protein-coupled receptors (GPCRs), and more particularly to non-peptidergic inverse agonists. Even more in detail, the present invention relates to non-peptidergic inverse agonists for a constitutively active viral-encoded G protein-coupled receptor, Further, the invention relates to the use of these agonists in viral pathogenesis.

Various viruses, and particularly herpes- and poxviruses, contain DNA sequences encoding proteins with homology to cellular chemokine receptors. These cellular chemokine receptors belong to the family of G protein-coupled receptors (GPCRS), Since GPCRs play a crucial role in cellular communication and chemokine receptors play a prominent role in the immune system, virally encoded GPCRs may be crucial determinants of viral action.

The β-herpesvirus human cytomegalovirus (HCMV) is a ubiquitous pathogen which causes severe diseases in immunocompromised individuals and has been suggested to play a role in atherosclerosis and arterial restenosia. HCMV encodes four GPCRs, among which US28 is particularly important with an eye on the present invention. This virally encoded GPCR US28 is able to induce migration of smooth muscle cells, a feature essential for the development of atherosclerosis. Moreover, US28 serves as a cofactor for the entry into cells of human immunodeficiency virus-type 1 (HIV.1).

More in detail, human cytomegalovirus (HCMV) is a widespread pathogen that does not cause significant clinical manifestations in healthy individuals. In contrast, however, primary infection or reactivation of the virus in immunocompromised hosts, such as AIDS patients, neonates, pregnant women, and patients on an immunosupressive drug regime, e.g. because of an organ transplantation, or with an otherwise suppressed immune system, such as patients undergoing anticancer therapies, can cause severe and even fatal disease. Moreover, a number of clinical studies have suggested that HCMV infection plays a role in the development of vascular diseases including vascular allograft rejection, restenosis and atherosclerosis. In this light, reference can be made to Sweet in FEMS Microbiol, Rev., 23 (1999), 457-482; Melnick in J. Med. Virol-, 42 (1994), 170-174 and Melnick et al. in Arch. Immunol. Thor. Exp., 44 (1996), 297-302.

HCMV encodes four putative GPRCs, namely US27, US28, UL33 and UL78. Like HCMV, various β- and γ-herpesviruses, such as human herpesviruses HHV-6, HHV-7 and HHV-8 (Kaposi's sarcoma associated Herpes virus) have been found to also encode GPRCs in their genome. These virally encoded GPRCs show homology to mammalian chemokine receptors, suggesting that these viruses exploit chemokine signaling pathways as a general mechanism to interfere with the host immune system. See in this respect, Murphy in Nat. Immunol, 2 (2001), 116-122.

As said, HCMV-encoded US28 shows high homology to chemokine receptors, such as CC-chemokine receptors. Yet, it also binds several other CC-chemokines such as CCL5/RANTES, CCL3/MIP-1α and CCL2/MCP-1 with high affinity.

Furthermore, US28 binds the membrane-bound CX3C-chemokme CX3CL1/fractalkine, which has been suggested to play a role in the cellular interaction with viral particles (Kledal *et al.*, FEBS Letters 441 (1998), 209-214). Similarly to the chemokine receptors CCR5 and CXCR4, US28 exhibits HIV cofactor activity when coexpressed with CD4 (Pleskoff *et al.*, Science 276 (1997), 1874-1878). Streblow *et al.* have shown in Cell 99 (1999), 511-520 that expression of US28 after infection with HCMV induces smooth muscle cells (SMCs) migration, providing a potential link between CMV and progression of vascular disease.

Gasarosa *et al.* have described in J. Biol. Chem. 276 (2) (2001), 1133-1137 that the HCMV-encoded GPCR US28 efficiently signals in an agonist-independent manner. US28 has been shown to constitutively couple to phospholipase C and NF-_{κ}B via related G_{q/11} protein-mediated mechanisms upon transient expression in COS-7 cells. US28 shows 33% homology to the CC chemokine receptor CCR1. This cellular homolog CCR1, however, does not display constitutive activity.

Constitutive activity of GPCRs occurs in the absence of neurotransmitter or hormone stimulation. The GPCR protein is thought to isomerise between at least two different states, in an inactive (R) and active (R*) conformation- Agonist binding is thought to shift the equilibrium toward R*, leading to an increased coupling to G proteins associated with an increase in GPCR activity, while inverse agonists inhibit constitutive GPCR activity. Neutral antagonists, on the other hand, do not modulate constitutive GPCR signaling.

The possible implications of constitutive activity of US28 in the pathogenesis of CMV infection have not been established yet. Also, other viral-encoded GPCRs, such as KSHV-encoded ORF74 (see Arvanitakis *et al.* in Nature 385 (1997) 347-350) and RCMV-encoded R33 (see Gruijthuijsen *et al.* in J. Virol. 76 (2002) 1328-1338), show constitutive activity. KSHV-encoded ORF74 provides some evidence for a link between constitutive activity and viral pathology. As described by Bais *et al.* in Nature 391 (1998) 86-89, ORF74 has oncogenic potential and transgenic mice expressing ORF74 develop angioproliferative lesions that morphologically resemble Kaposi Sarcoma (see also Yang *et al.* in J.Exp.Med. 191 (2000), 445-454).

In permissive cells, US28 is expressed as an early gene (Zipeto *et al.* J. Gen. Virol. 80 (1999), 543-547). Moreover, US28 mRNA can also be detected in semi- and non-permissive cells such as monocytes, which represent a typical site of latency for the virus. Different reports (Neote *et al.* Cell 72 (1993), 415-425; Gao & Murphy J. Biol. Chem. 269 (1994), 28539-28542; Kuhn *et al.* Biochem. Biophys. Res. Common. 211 (1995), 325-330) have shown that US28 binds several CC-chemokines such as CCL5/RANTES, CCL3/MIP-1 α, CCL4/MIP-1β and CCL2/MCP-1 with high affinity when expressed in HEK-293, K569 or COS-7 cells. US28 is reported to mediate an increase of intracellular calcium and activate p44/p42 Mitogen Activated Protein Kinases (MAPK.) in response to CC-chemokines (Gao & Murphy; J. Biol. Chem. 269 (1994), 28539-28542; Billstrom *et al.* J. Virol. 72(1998), 5535-5544). Similarly, Vieira et al. have shown in J. Virol. 72 (1998), 8158-8165 that fibroblasts infected with wild-type HCMV (WT-HCMV) bind CC-chemokines with high affinity and release intracellular calcium in response to them. In contrast, cells infected with a mutant virus in which US28 has been deleted do not show these features, demonstrating that 11528 is a functional receptor encoded by CMV. Furthermore, US28 can specifically recognize and bind the membrane-bound CX3C-chemokine fractalkine, which has been suggested to play a role in the interaction between virus and cell. See in this light, the above-mentioned article of Casarosa.

Similarly to other chemokine receptors, US28 exhibits HIV cofactor activity when coexpressed with CD4. In addition, it can enhance cell-cell fusion by a mechanism apparently distinct from HIV coreceptor activity (Pleskoff *et al.* J. Virol. 72 (1998), 6380-6397). It can be hypothesized that US28 plays a role in CMV entry, through its ability to enhance membrane fusion.

In the international patent application WO A2-02/17900, it is proposed to block CMV dissemination in a host by using a compound that inhibits the binding of a chemokine to US28 or a US28 fragment. By inhibiting dissemination of virus from sites of primary or recurrent infection, viral spread to secondary organs can be limited. This consequently limits viral replication. A large group of compounds is presented that is said to block CMV dissemination. In its broadest description this group of compounds have the following formula 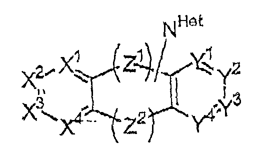 wherein
X¹, X², X³ and X⁴ are each independently members selected from the group consisting of N and C-R¹, wherein R¹ is a member selected from the group consisting of H, halogen, (C₁-C₄) alkyl, (C₁-C₄) alkoxy, (C₁-C₄) alkylthio, (C₁-C₄) haloalkyl, (C₁-C₄) haloalkoxy, nitro, cyano, (C₁-C₄) acyl, amino, (C₁-C₄) alkylamino, and di (C₁-C₄) alkylamino;
Y¹ ,Y², Y³ and Y⁴ are each independently members selected from the group consisting of N and C-R², wherein R² is a member selected from the group consisting of H, halogen, (C₁-C₄) alkyl, (C₁-C₄) alkoxy, (C₁-C₄) alkylthio, (C₁-C₄) haloalkyl, (C₁-C₄) haloalkoxy, nitro, cyano, (C₁-C₄) acyl, amino, (C₁-C₄) alkylamino, and di (C₁-C₄) alkylamino;
Z¹ represents a substituted or unsubstituted (C₁-C₃) alkylene;
Z² is a divalent moiety selected from the group consisting of -O-, -S- and -N(R³)-, wherein R³ is a member selected from the group consisting of H, halogen. (C₁-C₄) alkyl, (C₁-C₄) alkoxy, (C₁-C₄) haloalkyl, (C₁-C₄) haloalkoxy, nitro, cyano, (C₁-C₄) acyl, amino, (C₁-C₄) alkylamino, and di(C₁-C₄)alkylamino; and
N^{Het} is a substituted or unsubstituted 4-, 5-, 6-, or 7-membered nitrogen heterocycle,

WO-A-02/17969 also deals with compounds inhibiting chemokine binding at US28. Particularly, methods for blocking CMV dissemination in a host are described, which methods comprise the administration of an effective amount of a compound of the formula 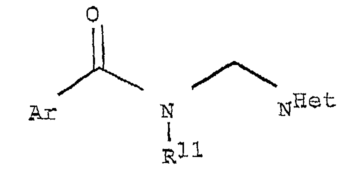 or a pharmaceutically acceptable salt thereof; wherein Ar represents a substituted aryl group; R¹¹ represents H or (C₁-C₄)alkyl; and N^{Het} is a substituted or unsubstituted 4-, 5- 6-, or 7-membered nitrogen heterocycle.

Preferred within this group are the compounds having the formula; 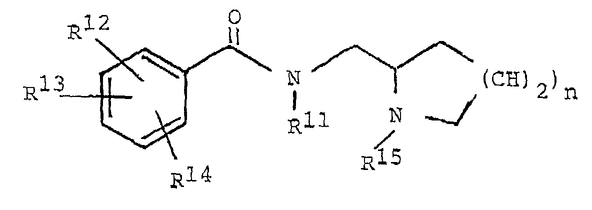 or a pharmaceutically acceptable salt thereof; wherein the subscript n is an integer of from 1 to 3; R¹¹ and R¹³ are independently selected from H and (C₁-C₄)alkyl; R¹², R¹³ and R¹⁴ are each members independently selected from H halogen, (C₁-C₄)alkyl, (C₁-C₄)alkoxy, (C₁-C₄)haloalkyl, (C₁-C₄)haloalkoxy nitro, cyano, (C₁-C₄)acyl, amino, (C₁-C₄)alkylamino, and di(C₁-C₄)alkylamino; with the proviso that at least one of R¹² , R¹³ and R¹⁴ is other than H.

In WO-A-00/11950 another mechanism is proposed. Cell migration initiation is prevented with CMV US28 receptor antagonists. Particularly it is taught to use KHSV encoded vMIP-2, fractalkaline or herbimycin.

So far all ligands reported to bind US28 belong to the family of chemokines.

It is an object of the present invention to find an inverse agonist that acts at US28. Such an inverse agonist would form an important tool to investigate the relevance of constitutive activity. An inverse agonist would inhibit receptor constitutive activity. In addition, it would act as an allosteric modulator of chemokinebinding at US28.

It is a further object to find small and preferably non-peptidergic ligands that bind US28.

A further object is to find antivirus agents, and particularly anti-HCMV and anti-HIV agents.

Other objects of the present invention will become clear after reading the detailed description herein-below.

Surprisingly, the present invention provides a group of compounds, which meet the objects set. Particularly the invention relates to a group of small, non-peptidergic compounds that bind US28, The group of compounds were found to act as inverse agonists at HCMV-encoded chemokine receptor US28. This binding to US28 was found specific in the sense that constitutive activity displayed by other viral GPCRs, ORF74 and R33 and the histamine H₁ receptor, which is also a G_{q} coupled receptor, is not affected by said compounds.

In a first aspect, the present invention relates to a non-peptidergic compound being an inverse agonist acting on HCMV encoded US28 receptor.

In a preferred embodiment, the compound of this invention has the structure of formula (I) 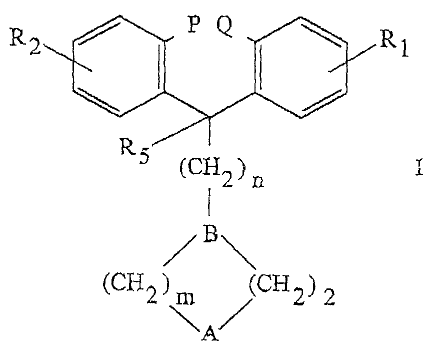 wherein:
A is selected from the group consisting of C(X)(Ar), N-(CH₂)ₖ-Y-Ar, 0, NH and N-R₄;
X is H, OH, OR₄, or R₄;
R₄ is, independently, a C₁₋₃ alkyl group, optionally substituted by inert substituents, such as one or more halogen atoms;
Ar is 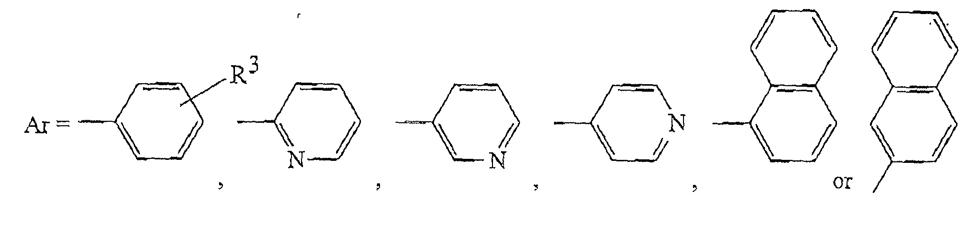
k is an integer of 0-6;
Y is C(OH)(H) or C=O;
B is CH or N;
with the proviso that when A is NH, B is not N;
m is an integer of 1-3;
n is an integer of 0-4;
R₅ is H, OH, CN, R₄, or CH₂NH₂;
R₁, R₂ and R₃, independently, represent H, OH, OR₄, a halogen atom, CN, a straight or branched C₁₋₅ alkyl that can optionally be substituted with one or more halogen atoms; and
P and Q represent each a hydrogen atom, or P and Q together are a -SCH₂-, a -CH₂S-, a -OCH₂-, a -CH₂O-, a -CH=CH- group or a C₁₋₂alkylene group.

Suitable examples of substituents R₁, R₂ and R₃ which are straight or branched C₁₋₅ alkyl are n-alkyl groups, secondary alkyl groups or tertiary alkyl groups, such as t-butyl groups. A suitable halogen substituted alkyl group for R₁, R₂ and R₃ is a perfluorosubstituted alkyl, such as a trifluoromethyl group.

Preferred compounds of the invention are compounds of the formula (I), wherein X is either a halogen atom and preferably a chlorine atom, or a hydroxyl group.

In a further embodiment, the present invention relates to the use of the compounds of the invention for the manufacture of a medicament having inverse agonist activity on HCMV encoded US28 receptor. Preferably, this medicament is used for or in the treatment of or for the prevention of HCMV infection. Since this is the first time that inverse agonists acting as US28 can be used in the inhibition of HIV entry into cells, the present invention also relates to the use of an inverse agonist acting at US28, in general, in the manufacture of a medicament for inhibiting or for the prevention of HIV entry into permissive cells. In a preferred embodiment, the medicament is a prophylacticum for an individual having a compromised immune system, or for an individual expected to encounter a suppressed immune system. Immunocompromised hosts which may have benefits from the present invention are AIDS patients, neonates, pregnant women, and patients on an immunosupressive drug regime, e.g. because of an organ transplantation, or with an otherwise suppressed immune system, such as patients undergoing anticancer therapy.

Another aspect of the present invention encompasses the use of any one of the compounds of the present invention in the manufacture of a medicament for inhibiting viral infections. Especially, medicaments to treat an HIV infection, such as in AIDS are preferred. In this embodiment, the compounds used in the manufacture of these medicaments can be combined with other antiviral agents which are either therapeutic or propylactic agents, or, alternatively or additionally, with agents that treat or induce conditions associated with the viral infection, such as anti HIV agents or immunosuppressive agents. So-called "combination therapy" in many instances enhances the efficacy of the agents of the invention, Exemplary antiviral agents include ganciclovir, foscarnet and cidofovir. Exemplary anti-HIV agents include indinavir, ritonavir, AZT, lamivudine and saquinavir. Exemplary immunosuppressive agents include cyclosporin, rapamycine and FK-506. Moreover, the medicaments made using the compounds of the invention can also suitably be combined with immunosuppressive protocols such as bone-marrow destruction, for instance by radiation therapy or chemotherapy.

The compounds of the invention are used in the manufacture of a medicament. Such a medicament comprises a pharmaceutically acceptable carrier or adjuvant and an effective amount of the compound of the invention.

Typically, the compositions contain form about 0,1% to about 99% by weight of active compound, and preferably from about 10% to about 60% by weight depending on which method of administration is employed.

For the compositions of the invention, the proportion of each carrier, diluent or adjuvant is determined by the solubility and chemical nature of the compound and the route of adminstration according to standard pharmaceutical practice. In order to obtain consistency of administration, however, it is preferred that a composition of the invention is in the form of a unit dose. For example, the unit dose presentation forms for oral administration may be tablets and capsules and may contain conventional excipients such as binding agents (e.g., acacia, gelatin, sorbitol, or polyvinylpyrrolidone), fillers (e.g. lactose, sugar, maize-starch, calcium phosphate, sorbitol or glycine) tableting lubricants (e.g. magnesium stearate), disintegrants (e.g. starch, polyvinylpyrrolidone, sodium starch glycoallate or microcrystalline cellulose), or pharmaceutically acceptable wetting agents (e.g., sodium lauryl sulfate).

The compounds may be injected parenterally; this being intramuscularly, intravenously, or subcutaneously. For parenteral administration, the compound may be used in the form of sterile solutions containing other solutes, for example, sufficient saline or glucose to make the solution isotonic. The amount of active ingredient adminstered parenterally will be approximately 0.01 to 250 mg/kg/day, preferably about 1 to 10 mg/kg/day, more preferably about 0.5 to 30 mg/kg/day, and more most preferably about 1-20 mg/kg/day.

The compounds may be administered orally in the form of tablets, capsules, or granules containing suitable excipients such as starch, lactose, white sugar and the like. The compounds may be adminstered orally in the form of solutions which may contain coloring and/or flavoring agents. The compounds may also be administered sublingually in the form of tracheas or lozenges in which each active ingredient is mixed with sugar or corn syrup, flavoring agents and dyes, and then dehydrated sufficiently to make the mixture suitable for pressing into solid form. The amount of active ingredient adminstered orally will depend on bioavailability of the specific compound.

The solid oral compositions may be prepared by conventional methods of blending, tableting, or the like. Repeated blending operations may be used to distribute the active agent throughout those compositions employing large quantities of tillers. Such operations are, of course, conventional in the art. The tablets may be coated according to methods well known in normal pharmaceutical practice, in particular with an enteric coating.

Oral liquid preparations may be in the form of emulsions, syrups, or elixers, or may be presented as a dry product for reconstitution with water or other suitable vehicle before use. Such liquid preparations may or may not contain conventional additives. For example suspending agents, such as sorbitol, syrup, methyl cellulose, gelatin, hydroxyethyl cellulose, carboxymethylcellulose, aluminum stearate gel, or hydrogenated edible fats; emulsifying agent, such as sorbiton monooleate or acaci; non-aqueous vehicles (which may include edible oils), such as almond oil, fractionated coconut oil, oily esters selected from the group consisting of glycerin, propylene glycol, ethylene glycol, and ethyl alcohol; preservatives, for instance methyl parahydroxybenzoate, ethyl parahydroxybenzoate, n-propyl parahydroxybenzoate, or n-butyl parahydroxybenzoate of sorbic acid; and, if desired, conventional flavoring or coloring agents.

The compounds of the present invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions etc., containing the compounds of the present invention are employed. As used herein, topical application is also meant to include the use of mouth washes and gargles.

When used as a medicament, the compounds of the present invention give good results when they are administered in amounts of about 0.01-250 mg/kg body weigth/day, preferably about 1 to 10 mg/kg/day, more preferably about 0.5-30 mg/kg/day, and most preferably in an amount of about 1-20 mg/kg/day.

The compounds of the invention can also be present in the form of their pharmaceutically acceptable salts.

The term "pharmaceutically acceptable salts" is meant to include salts of the active compounds which are prepared with relatively nontoxic acids or bases, depending on the particular substituents found on the compounds described herein. When compounds of the present invention contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When compounds of the present invention contain relatively basic functionalities, acid addition salts can be obtained by contacting the neutral form of such compounds with a sufficient amount of the desired acid, either near or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric sulfuric, monohydrogensulfuric, hydriodic, or phosphorous acids and the like, as well as the salts derived from relatively nontoxic organic acids like acetic, propionic, isobutyric, maleic, malonic, benzoic, succinic, suberic, fumaric, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like (see, for example, Berge, S.M., et al. "Pharmaceutical Salts", Journal of Pharmaceutical Science, 1977, 66, 1-19). Certain specific compounds of the present invention contain both basic and acidic functionalities that allow the compounds to be converted into either base or acid additional salts.

The neutral forms of the compounds may be regenerated by contacting the salt with a base or acid and isolating the parent compound in the conventional manner. The parent form of the compound differs from the various salt forms in certain physical properties, such as solubility in polar solvents, but otherwise the salts are equivalent to the parent form of the compound for the purposes of the present invention.

Certain compounds of the present invention can exist in unsolvated forms as well as solvated forms, including hydrated forms. In general, the solvated forms are equivalent to unsolvated forms and are intended to be encompassed within the scope of the present invention. Certain compounds of the present invention may exist in multiple crystalline or amorphous forms. In general, all physical forms are equivalent for the uses contemplated by the present invention and are intended to be within the scope of the present invention.

Certain compounds of the present invention possess asymmetric carbon atoms (optical centers) or double bonds; the racemates, diastereomers, geometric isomers and individual isomers are all intended to be encompassed within the scope of the present invention.

Further, the invention relates to the use of a compound according to the invention in the manufacture of a diagnosticum for detecting or imaging CMV infection in a host, which diagnosticum is intended to be administered to a host suspected to have a CMV infection or to a sample taken from said host, after which binding of said compound in the host or sample is measured. Particularly, the compound of the invention can for this embodiment be labelled, for instance by using a suitable radioisotope.

In yet another aspect, the present invention relates to an assay using constitutively active virus-encoded GPCRs as screening method of non-peptidergic, drug-like ligands for these (orphan) receptors. Details of this assay are given herein-below.

The invention in its current best mode relates to the compound having formula X: 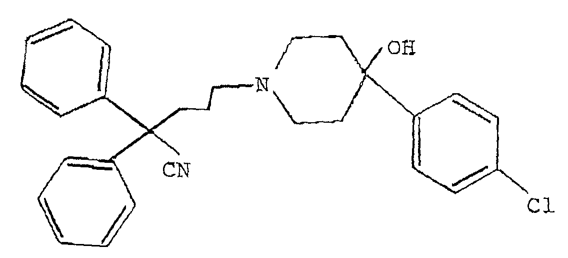 for use as an inverse agonist acting at US28 receptor.

This compound having formula X has previously been described by Hesselgesser *et al.* in J.Biol Chem. 273 (1998), 15687-15692, as an antagonist for the human chemokine receptor CCR1, which shares 30% homology with US28.

The present invention will now be described in more detail with a focus on the preferred embodiment, the compound of Formula X.

In this detailed description, reference will be made to the drawings, showing:

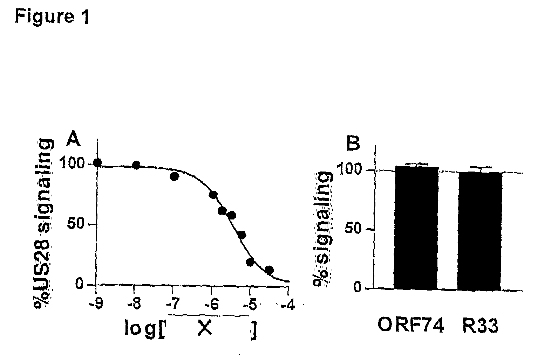

Fig. 1: Inhibition of US28-mediated inositol phosphates accumulation by the compound of Formula X.
Fig 1A: COS-7 cells expressing US28 were incubated with various concentrations of the compound of Formula X and InsP release was measured. Data are presented as percentage of US28-mediated response, defined as absolute increase of US28-mediated InsP accumulation above values obtained for mock-transfected cells. The average of 11 experiments, with each data point performed in triplicate, is shown.
Fig 1B: ORF74- and R33-transfected COS-7 cells were incubated with the compound of Formula X (10 µM) and InsP accumulation was measured. Data are presented as percentage of receptors' basal signaling. The average of 3 experiments, with each data point performed in triplicate, is shown.

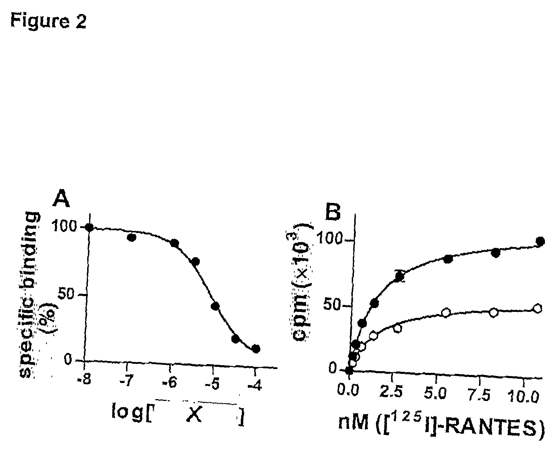

Fig. 2: Displacement of [¹²⁵I]-RANTES binding at US28 by the compound of Formula X.
Fig 2A: COS-7 cells expressing US28 were incubated with [¹²⁵I]-RANTES (filled circles) in the presence of various concentrations of the compound of Formula X. Data are presented as percentage of US28 specific binding. The average of 4 experiments, with each data point performed in triplicate, is shown.
Fig 2 B: US28-transfected COS-7 cells were incubated with several concentrations of [¹²⁵I]-RANTES in the presence (open circles) or absence (filled circles) of the compound of Formula X (10 µM). Data are presented as specific binding at US28 (cpm). A representative experiment out of three experiments, each performed in triplicate is shown.

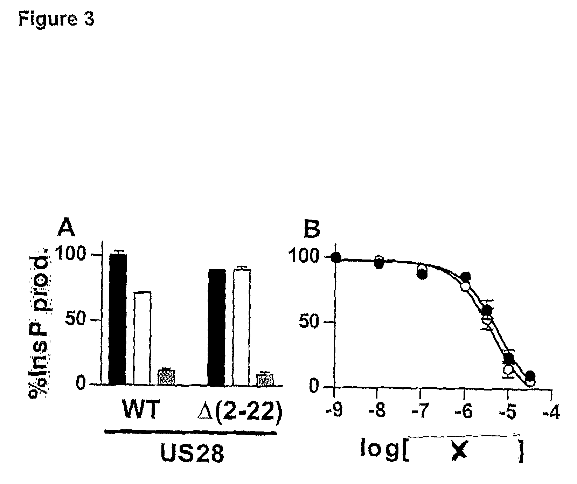

Figure 3. Role of the N-terminus of US28 for the action of inverse agonists.
Fig 3A: COS-7 cells expressing either WT- or Δ(2-22)-US28 were assayed for InsP accumulation in the presence or absence (filled bars) of fractalkine (100 nM, open bars), the compound of Formula X (10 µM, grey bars) or medium alone (black bars), Data are presented as percentage of WT-US28 mediated response. The average of 3 experiments, with each data point performed in triplicate, is shown.
Fig 3B: COS-7 cells expressing either WT- (open circles) or Δ(2-22)-US28 (filled circles) were assayed for InsP accumulation in the presence of different concentrations of the compound of Formula X. Data are presented as percentage of receptors' mediated response, The average of 3 experiments, with each data point performed in triplicate, is shown.

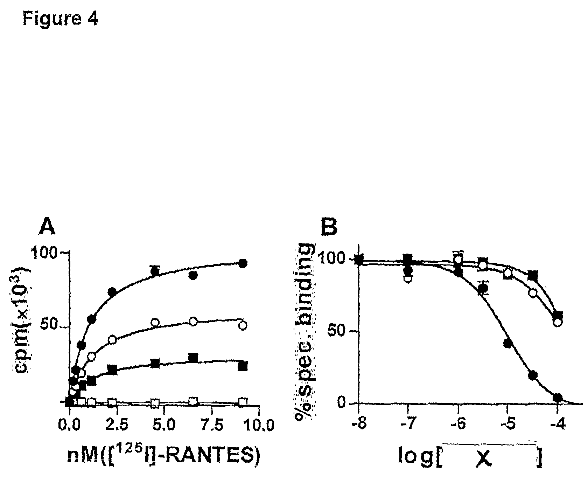

Fig. 4: Binding properties of [¹²⁵I]-RANTES and the compound of Formula X at mutant and WT-US28 receptors.
Fig 4A: Saturation binding with [¹²⁵I]-RANTES was performed on COS-7 cells expressing either Δ(2-22)- (open squares), E²⁷⁷A- (filled squares), E²⁷⁷Q- (open circles) or WT-US28 (filled circles). Data are presented as specific binding (cpm). A representative experiment out of three experiments, each performed in triplicate is shown.
Fig 4B: COS-7 cells expressing either E²⁷⁷A- (filled squares), E²⁷⁷Q-(open circles) or WT-US28 (closed circles) were incubated with [¹²⁵I]-RANTES in the presence of various concentrations of the compound of Formula X. Data are presented as percentage of receptors' specific binding. The average of 3 experiments, with each data point performed in triplicate, is shown.

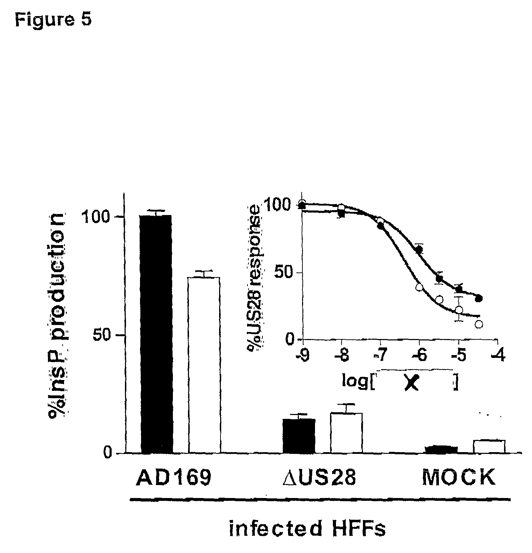

Fig. 5: US28 mediates PLC activation in HCMV-infected fibroblasts. HFFs were either mock-, WT-HCMV- (strain AD169) or Δ28-HCMV-infected and after 48 hours were assayed for InsP accumulation in the presence (open bars) or absence (filled bars) of fractalkine (100 nM), The average of 2 experiments, with each data point performed in triplicate, is shown. Inset: HCMV-infected HFFs were assayed for InsP accumulation (filled circles) or [¹²⁵I]-RANTES binding (open circles) in the presence of different concentrations of the compound of Formula X. Data are presented as percentage of HCMV-mediated response, defined as absolute increase of InsP accumulation or [¹²⁵I]-RANTES binding above mock-infected cells. The average of 3 experiments, with each data point performed in triplicate, is shown.

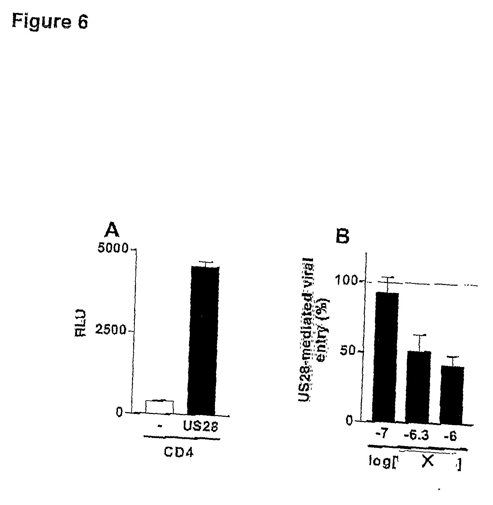

Fig. 6: Inhibition of 11528-mediated HIV-1 entry by the compound of Formula X.
Fig 6A: HEK293-T cells were cotransfected with CD4 and US28 or CD4 alone and infected with the luciferase-containing HIV-1 reporter virus. Data are presented as relative light units (RLU). The average of 3 experiments, with each data point performed in triplicate, is shown.
Fig 6B: HEK293-T cells co-transfected with CD4 and US28 were incubated with different concentrations of the compound of Formula X or DMSO (negative control) and infected with the luciferase-containing HIV-1 reporter virus. Data are presented as percentage of US28 co-receptor activity, defined as absolute increase of US28-mediated viral entry above values obtained for cells transfected with CD4 alone. The average of 2 experiments, with each data point performed in triplicate, is shown.

### Materials and Methods

DNA constructs. The cDNA encoding for US28 (see Casarosa *et al.* in J, Biol. Chem. 276 (2001) 1133-1137) was inserted into the mammalian vector pcDNAS. Δ(2-22)-US28 and the HA-tagged versions of both WT- and Δ(2-22)-US28 were generated by PCR. Single amino acid mutations, as for E²⁷⁷A- and E²⁷⁷Q.US28, were introduced using the Altered Sites® II *in vitro* Mutagenesis System (Promega, Madison, USA), according to manufacturer's protocol. All constructs were verified by dideoxy sequencing.

Synthesis of compounds. The compound of Formula X and analogues were synthesized following the methods published by Hesselgesser *et al.* in J. Biol. Chem. 273 (1998) 15687-15692. The structures were confirmed by NMR analysis. Other compounds of the present invention can be synthesized while using the methods described in WO-A-98/33789

Cell culture, transfection and infection with HCMV. COS-7 cells were grown as previously described in the cited Casarosa reference. Transfection of the COS-7 cells was performed by DEAE-dextran, using 2 µg of DNA of each US28 construct per million cells. Human foreskin fibroblasts (HFFs) were cultured and infected with WT-RCMV (strain AD169) or a deletion mutant Δ28-HCMV.

[³H]-inositol phosphate production. Experiments in COS-7 cells and HFFs were performed as described in the references mentioned in the previous paragraph.

Binding experimants. Labeling of RANTES (Peprotech, Rocky Hill, USA) with [¹²⁵I] and binding in COS-7 cells were performed as previously described (Gruijthuijsen *et al.*, J. Virol. 76 (2002) 1328-1338).

HIV-1 infection assay. HEK293-T cells were transiently transfected with pcDNA1-US28 and pcDNA1-CD4 using the calcium phosphate method and cultured overnight in 48 well plates. The following day, cells were incubated with different concentrations of the compound of Formula X (dissolved in DMSO) or medium (containing an equal amount of DMSO) for 2 hours before infection with an R5-tropic HIV- 1 virus containing the luciferase reporter gene under the LTR promoter. After overnight incubation, cells were washed twice and cultivated in fresh medium. 3 days after infection, the luciferase activity was quantitated using the Luciferase Assay System (Promega, Madison, USA).

ELISA. 48 hours after transfection, receptors' expression in COS-7 cells was measured with an ELISA assay as described by Gruijthuijsen *et al.*, in J. Virol. 76 (2002) 1328-1338. Mouse anti-HA monoclonal antibody (provided by Dr. J, van Minnen, Vrije Universiteit Amsterdam, The Netherlands) was used as primary antibody, and goat anti-mouse-horseradish peroxidase conjugate (Bio-Rad, Hercules, USA) as secondary antibody. TMB solution (Sigma) was used as substrate and the optical density was measured in a Victor² (PerkinElmer, Boston, USA) at 450 nm,

Toxicity test. The AlamarBlue^{TM} assay (Serotec, Oxford, UK) was performed following the manufacturer's protocol. Briefly, transfected cells were incubated with the compound of Formula X for 2 hours, followed by the addiction of the AlamarBlue dye.

After 1 hour, the fluorescence was monitored at 560 nm excitation wavelength and 590 nm emission wavelenght in a Victor² (PerkinElmer, Boston, USA).

Generation of *in silico* model of US28. An alignment was made between bovine rhodopsin and US28 using Clustal X. A homology model of US28 was generated using the homology module of Insight II, version 2.3.0 (Biosym Technologies, San Diego, USA). The A chain from the crystal structure of bovine rhodopsin was used as a template (see in this respect Okada *et al.* in J. Struct. Biol. 130 (2000) 73-80).

Data analysis. Curve fitting of data was carried out using the program Prism and IC₅₀ values obtained by nonlinear regression analysis (GraphPad Software, Inc., San Diego, USA). Data are expressed as mean ± S.E.

### Detailed description of the invention

In order to find inverse agonists for US28, a variety of compounds were screened for modulation of US28-mediated constitutive inositol phosphate (InsP) production. This approach led to identify the compound of Formula X as inverse agonist for US28.

The compound of Formula X dose-dependently inhibits US28 mediated InsP production in COS-7 cells (fig. 1A). As the observed inhibition of US28 signaling is complete, the compound of Formula X behaves as a full inverse agonist with an IC₅₀ of 3.5 µM (pIC₅₀= 5.46 ± 0.07). The compound of Formula X did not show cellular toxicity as determined with the AlamarBlue^{TM} assay (data not shown).

Moreover, other GPCRs were used as controls to ascertain specificity of action of this compound. As previously reported, expression ofRCMV-encoded R33 (Gruijthuijsen *et al.* J. Virol. 76 (2002), 1328-1338) or KSHV-encoded ORF74 (Rosenkilde *et al.* 274 (1999), 956-961) also leads to constitutive activation of phospholipase C. The compound of Formula X does not modulate R33- or ORF74-mediated PLC activation at concentration as high as 10 µM (fig. 1B).

Furthermore, the compound of Formula X was tested on COS-7 cells expressing the human histamine H₁ receptor (hH₁). Like US28, this GPCR constitutively activates PLC enzymes via Gα_{q/11} proteins. The compound of Formula X (10 µM) does not alter basal or histamine-induced production of InsP in COS-7 cells expressing the H₁ receptor, ruling out a possible interference with Gα_{q/11} proteins,

Several analogues of the compound of Formula X were subsequently synthesized in order to unravel structural features important for its inverse agonistic effect at US28 (Table 1).
Table 1 Activities of various VUF2274 analogues, VUF2274 analogues were tested for their ability to inhibit US28-mediated InsP production or [¹²⁵I]-RANTES binding Values are expressed in µM and represent the average of at least two independent experiments. S.E. calculated on the pIC₅₀'s is within ± 0.2.

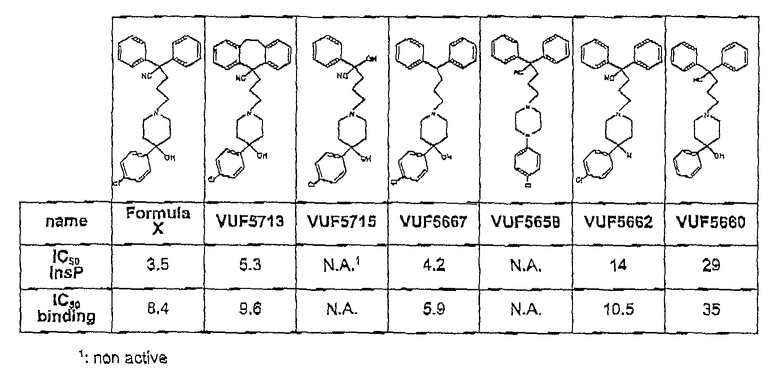

Variations of the benzhydryl moiety are well allowed: insertion of an ethylene (VUF5713) or a thiomethylene bridge results in compounds of comparable potencies, indicating that some flexibility is tolerated in this part of the template. Suitable bridges for P and Q in Formula I include -OCH₂-, -CH₂O-, -SCH₂-, -CH₂S-.

On the other hand, replacement of one of the phenyl rings with more hydrophilic moieties, such as a hydroxyl group (VUF5715) results in a loss of effect, suggesting that a bulky lipophilic moiety is important. The nitrite substituent does not appear to play a critical role; its replacement by a hydrogen atom (see VUF5667) does not affect the activity of the compound. This means that any substituent that does not considerably affect the configuration of the total compound can be used as a candidate to replace substituent R₅ group in formula I. In a preferred embodiment, R₅ in Formula I represents CN.

Replacement of the piperidine ring with a piperazine ring (VUF5658) results in an inactive compound.

Further, removal of the hydroxyl group (VUF5662) or the chlorine substituent (VUF5660) is not preferred in respect of the inverse agonistic potency; it results in a five- to ten-fold loss in potency.

In a preferred embodiment, group X in formula I is a halogen atom or a hydroxyl group.

The following tables show compounds which were tested on inverse agonist activity on US28:

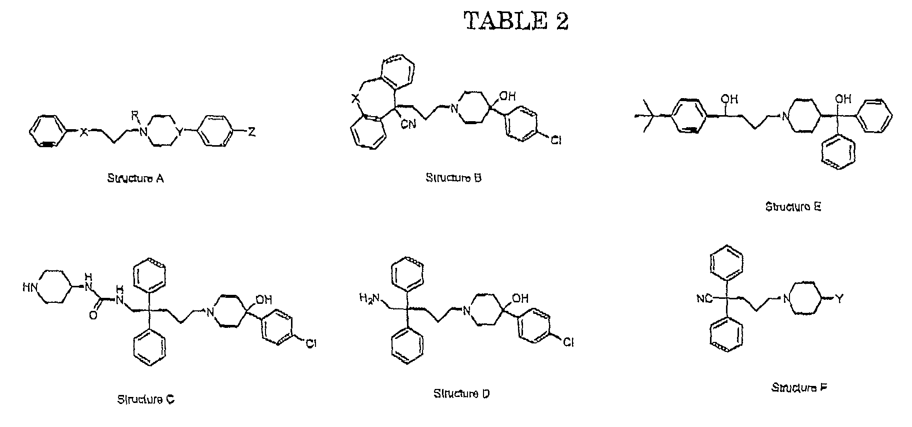

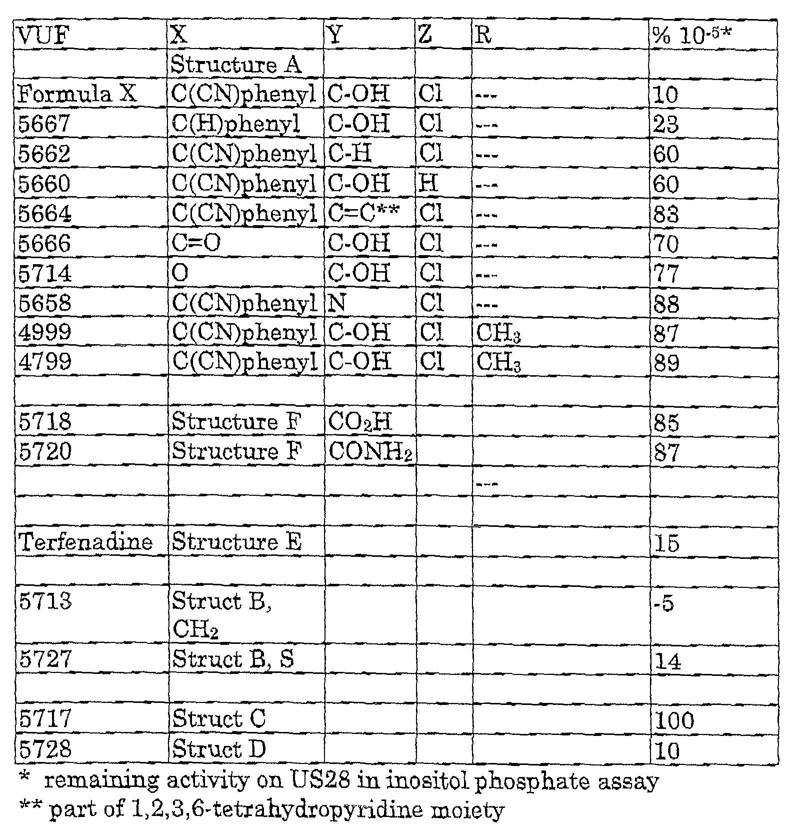

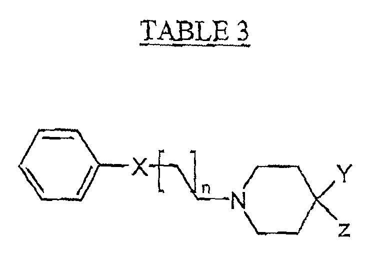

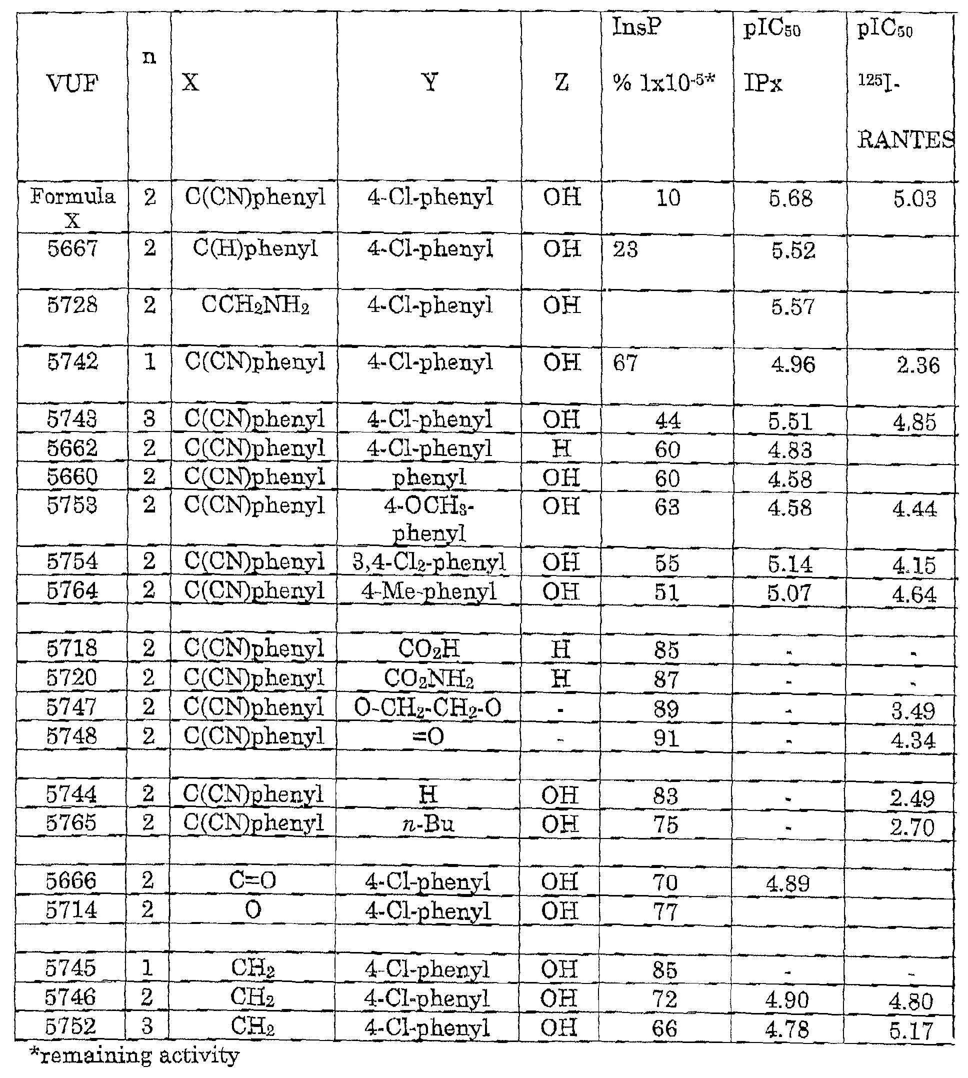

In EP-A-0 639 976, terfenadine, α-[4-(1,1-dimethylethyl)phenyl]-4-(hydroxydiphenylmethyl)-1-piperidinebutanol, is described as a known antihistaminic agent. Derivatives of this known compound (which falls within the definition of the compounds of Formula I) are described as antihistaminics in hepatically impaired patients.

US-A-5,925,761 describes the preparation of terfenadine and its derivatives. On the basis of this teaching, compounds of the present invention can also be synthesized in analogous ways.

WO 97/24325 describes diphenyl methane derivatives of the formula C(Ar₁)(Ar₂)(Q¹-X)(Q²-Z)
wherein Ar₁ and Ar₂ are, independently an optionally substituted aromatic group; Q¹ and Q² independently represent an optionally substituted divalent C₁₋₆ aliphatic hydrocarbon group, optionally having an oxygen or sulfur atom in the carbon chain;
X is an amino group, or wherein the hydrogen atoms can be replaced by optionally substituted lower alkyls, or lower alkyl-carbonyls or an acyl; or X can be a nitrogen containing heterocyclic ring; and
Z is an optionally substituted monocyclic or fused nitrogen-containing heterocyclic group. These compounds are said to be MIP-1α/RANTES receptor antagonists and as such useful for preventing or treating allergic diseases, like bronchial asthma, atopic dermatitis; inflammatory diseases of the type of arteriosclerosis and rheumatoic arthritis; and multiple sclerosis. The compounds of the present invention are not disclosed.

In WO-01/09094, WO-O1/09137 and WO-01/09138 chemokine receptor antagonists are described for treating aberrant leukocyte recruitment andlor activation. These antagonists are represented by the formula 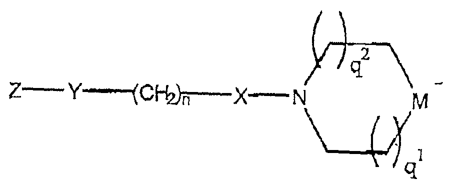 wherein:
Y is a single covelent bond;
n is an integer from one to about four;
X is a single covalent bond;
M is >NR², >CR¹R², - O-CR¹R²-O- or -CH₂-CR¹R²-O-;
The ring containing M is substituted or unsubstituted;
q¹ is an integer, such as an integer from zero to about three;
q² is an integer from zero to about one;
R¹ is -H, -OH, -N₃, a halogen, an aliphatic group, -O- (aliphatic group), -O- (substituted aliphatic group), -SH, -S- (aliphatic group), -S- (substituted aliphatic group), -OC(O)-(aliphatic group), -O-C(O)- (substituted aliphatic group),
   -C(O)O-(aliphatic group), -C(O)O-(substituted aliphatic group), -COOH, -CN, -CO-NR³R^{4,} -NR³R⁴ or R¹ is a covalent,
R¹¹ and R¹², taken together with the nitrogen atom to which they are bonded, form a non-aromatic heterocyclic ring;
X₂ is -S-CH₂-, -CH₂-S-, -CH₂-O-, -O-CH₂-, -CO-NR_{c}-, -NR_{c}-CO-, - CH₂-S(O)₂-, S(O)₂-CH₂-, -CH₂-NR_{c}-, NR_{c}-CH₂-, -CH₂-CH₂-, -CH=CH-, -CH₂-SO-, -SO-CH₂-, -O- or a bond;
R_{c} is hydrogen, an aliphatic group, a substituted aliphatic group, an aromatic group, a substituted aromatic group, a benzyl group or a substituted benzyl group, and
Ring A and Ring B are independently substituted or unsubstituted.

In further investigations, the present inventors have displaced chemokine binding at US 28 It was found that the compound of Formula X dose-dependently displaces [¹²⁵I]-RANTES binding to US28 in transiently transfected COS-7 cells with an IC₅₀ of 8.4 µM (pIC₅₀= 5,07 ± 0.1; fig. 2A). Various analogs of the compound of Formula X were also tested in [¹²⁵I]-RANTES displacement (see Table 1). In general, affinity for US28 correlates well with the potency of these compounds in the InsP assay.

Moreover, the present inventors investigated the effect of the compound of Formula X in a saturation binding assay of [¹²⁵I]-RANTES. In the presence of the compound of Formula X, the Bₘₐₓ value of [¹²⁵I]-RANTES is markedly decreased (Bₘₐₓ = 725 ± 69 versus 380 ± 36 fmol/mg protein in the absence and presence of 10 µM the compound of Formula X, respectively; fig.2B), while the K_{d} is barely affected (pK_{d}= 8.85 ± 0.01 and 8.78 ± 0.04 in the absence and presence of the compound of Formula X, respectively), suggesting that compounds having Formula I in general and the compound of Formula X in particular act as noncompetitive inhibitor.

In order to study which structural elements of US28 are responsible for interaction with chemokines and - as an example of the group compounds having Formula I - the compound of Formula X, different mutant receptors were generated. The extracellular N-terminal domain of chemokine receptors is generally thought to play an important role in binding of chemokines (see, e.g. Gayle *et al.* in J. Biol. Chem. 268 (1993), 7283-7289; Monteclaro & Charo in J. Biol. Chem. 272 (1997), 23186-23190; and Pease *et* al. in J. Biol. Chem. 273 (1998), 19972-19976). In order to elucidate the role of the N-terminus of US28 in chemokine binding, a mutant US28 receptor, which lacks the first 22 amino acids of the N-terminus (referred to as Δ(2-22)-US28) was generated. To measure receptor expression in transfected cells, the WT- and Δ(2-22)-US28 receptors were epitope-tagged at their N-terminus. The HA-epitope was found not to alter WT-US28 receptor expression, signaling or chemokines binding.

The N-terminus deletion mutant is expressed at the cell surface (39 ± 4% compared to WT-US28, n=3) as determined by ELISA. However, Δ(2-22)-US28 does not bind any of the tested chemokines ([¹²⁵I]-RANTES, fig. 4A, or [¹²⁵I]-fractalkine, data not shown) at concentration as high as 10 nM. Interestingly, Δ(2-22)-US28 still shows constitutive production of InsP (fig. 3A), proving that truncation of the N-terminus does not affect the correct orientation of the receptor macromolecule at the cell surface. In contrast to WT-US28, Δ(2-22)-US28 basal signaling is not inhibited by the CX3C-chemokine fractalkine (fig. 3A), again indicating the loss of chemokine binding for this mutant. Yet, the compound of Formula X totally inhibits the constitutive signaling of Δ(2-22)-US28 with a similar potency as observed for WT-US28 (pIC₅₀= 5.35 ± 0.1; fig. 3B).

In order to characterize the binding site of the compounds of Formula I, an *in silico* model of US28 was generated based on homology with bovine rhodopsin (see in this respect Okada *et al*. in J. Struct. Biol. 130 (2000), 73-80). Small non-peptidergic ligands are known to usually bind within the 7TM domains of GPCRs (Wieland *et al.,* J. Biol. Chem. 274 (1999), 29994-30000); Dragic *et al.,* Proc. Natl. Acad. Sc. USA 97 (2000), 5639-5644). The inventors focused on for negatively charged amino acids which would be in the hydrophilic pocket between the 7TM domains as potential interaction partners for the basic nitrogen of the piperidine moiety of the compounds of Formula I, which is predominantly protonated at physiological pH.

A glutamic acid residue in TM7 (E²⁷⁷) appeared an interesting candidate for its potential water accessibility. E²⁷⁷ was therefore mutated into glutamine, to eliminate the charge but retain the hydrogen bonding potential of the side chain, and into alanine, to eliminate both the charge and the hydrogen bonding potential. E²⁷⁷A- and E²⁷⁷Q-US28 are expressed at the membrane surface of COS-7 cells (Bₘₐₓ = 663 ± 49; 421 ± 45 and 285 ± 57 fmol/mg protein for WT-, E²⁷⁷A and E²⁷⁷Q-US28, respectively; fig. 4A) and bind [¹²⁵I]-RANTES with an affinity comparable to WT-US28 (pK_{d}, = 8.85 ± 0.1; 8.89 ± 0.1 and 8.90 ± 0.1 for WT-, E²⁷⁷A and E²⁷⁷Q-US28, respectively; fig. 4A), showing that the glutamic acid residue does not play a critical role in receptor-chemokine interaction. Interestingly, the affinity of the compound of Formula X is markedly reduced for both these mutants when compared to WT-US28 (fig. 4B). These results imply that the glutamate in position 277 indeed provides part of the interaction site for the compound of Formula X, probably via an ion-pair interaction.

The present inventors have also found that the compound of Formula X inhibits US28-mediated signaling in HCMV-infected fibroblasts.

It has been reported that infection of human foreskin fibroblasts (HFFs) with HCMV (strain AD 169) induces a consistent increase in PLC activity.

In order to investigate the role of HCMV-encoded GPCR US28 in this process, a deletion mutant virus (referred to as HCMV-AUS28), in which the open reading frame encoding US28 has been disrupted, was generated. Figure 5 shows the InsP turnover in either mock-, WT- (strain AD169) or HCMV-AUS28-infected HFFs. US28 expression is mainly responsible for constitutive activation of PLC, since in HFFs infected with HCMV-AUS28 inositol phosphate levels are dramatically reduced (10.6 ± 1.5% of WT-HCMV induced InsP). The CX3C-chemokine fractalkine partially inhibits (28 ± 4 %) the InsP production in CMV-infected cells (fig. 5), while it does not affect the InsP signaling in HCMV-AUS28- or mock-infected HFFs. As observed with COS-7 cells, the compound of Formula X dose-dependently inhibits US28 mediated signaling (inset fig. 5) in AD 169-infected HFFs with an IC₅₀ of 776 nM (pIC₅₀= 6,11 ± 0.04), while the ligand does not affect InsP levels in mock- or HCMV-ΔU28-infected cells. As previously reported by Vieira *et al.* in J. Virol. 72 (1998), 8158-8165, US28 expressed in AD169-infected HFFs binds [¹²⁵I]-RANTES with high affinity. In competition binding studies the compound of Formula X completely displaces [¹²⁵I]-RANTES with an IC₅₀ of 708 nM (pIC₅₀= 6.15 ± 0.1; inset fig.3).

In yet another test set-up, the present inventors have shown that the compound of Formula X inhibits US28-mediated HTV-1 entry.

US28 has been shown to be a broadly permissive co-receptor for HIV-1 when expressed in the presence of CD4 (see Pleskoff *et al.* in Science 276 (1997) 1874-1878). In order to test the ability of the compound of Formula X to block viral entry via US28, a reporter gene assay was used. In this assay, HEK-293T cells expressing US28 and CD4 are infected by a luciferase-containing HIV-1 reporter virus. Consequently, the level of cellular luciferase activity is proportional to HIV-1 entry. Results obtained with this assay confirm that US28 shows HIV-1 co-receptor properties. Luciferase activity is increased approximately 20 fold in the presence of US28 when compared to cells expressing CD4 alone (fig. 6A). The compound of Formula X was tested at several concentrations and it showed a dose-dependent inhibitory effect of US28-mediated viral entry (fig. 6B). At 10⁻⁶M, the compound of Formula X reduces HIV-1 entry to 41 ± 8 % of control cells. Determination of a complete dose-response curve was not possible due to toxic effects of DM50 and the compound at higher concentrations, as determined by MTT test.

Using the US28-mediated constitutive activation of PLC as screening approach, the present inventors have identified a group of molecules including the small non-peptidergic molecule of Formula X as inverse agonists at the HCMV-encoded chemokine receptor US28. Possible interferences of the compound of Formula X with G proteins or other downstream components in the InsP signaling cascade were ruled out using different GPCRs as controls. Several analogues of the compound of Formula X were subsequently tested. Several emerging points are noteworthy: the piperidine ring as well as the hydroxyl group and the chloro substituent at the phenyl ring represent important moieties for the activity of the compound of Formula X. On the other hand, more degree of freedom is tolerated at the benzhydryl moiety, where an increase in the lyphophilicity or the removal of the cyano group is well accepted.

The compound of Formula X dose-dependently displaces [¹²⁵I]-RANTES binding to US28, apparently with a noncompetitive behaviour, as shown by the marked decrease of the Bₘₐₓ value of [¹²⁵I]-RANTES in the presence of the compound of Formula X. Chemokines are relatively large peptides which interact mainly with the extracellular part of the receptor, such as the N-terminus and extracellular loops. On the other hand, small non-peptidergic compounds typically have interaction points located in the TM domains of GPCRs In fact, different studies have shown that non-peptide antagonists for peptide receptors, such as the glucagon receptor, and the chemokine receptor CCR3, often act as allosteric antagonists and show a noncompetitive behavior. Similarly, mutational analysis performed on US28 suggests that chemokines and the compound of Formula X bind to different receptor's epitopes. In fact, the N-terminus deletion mutant Δ(2-22)-US28 does not bind any of the tested chemokines, while its constitutive signaling is still inhibited by the compound of Formula X with a similar potency as observed for WT-US28.

Taken together, these data prove that the N-terminus of US28 is a determinant for chemokine binding but not for the small compound the compound of Formula X. Without wishing to be bound by any theory, it is believed that these results, together with the noncompetitive behaviour shown in saturation binding assays, suggest that displacement of the CC-chemokine RANTES is the result of a change in the conformation of the receptor allosterically induced by the compounds of the invention, and especially the compound of Formula X, and not due to competition for the same binding site.

Mutation of the glutamic acid residue E²⁷⁷ in TM7 of US28 showed that this residue is not important for RANTES binding, whereas it is a crucial interaction partner for the compound of Formula X, possibly through an ion-pair interaction with the basic nitrogen of the piperidine moiety. Interestingly, a glutamic acid residue is highly conserved in this position within the seventh transmembrane domain of chemokine receptors, whereas acidic residues are rare in this position in other GPCRs.

In order to study the action of the compound of Formula X on viral activity, the effect of the compound of Formula X in HCMV-infected fibroblasts was investigated. HCMV infection is accompanied by activation of several signaling pathways in infected cells, among which a US28-dependent increase in the intracellular levels of inositol phosphates. This model offers advantages for the pharmacological study of US28, since it closely resembles the patho-physiological situation. In this condition, US28 expression is regulated by the virus and its constitutive signaling is not a potential artifact due to over-expression, Moreover, HCMV might encode some signaling partner(s) for US28 which could alter US28 behaviour and that would not be present in the transfected system. In line with data obtained in COS-7 cells, the compound of Formula X dose-dependently inhibited US28 mediated signaling in HCMV-infected fibroblasts. These results confirm the action of the compound of Formula X as an inverse agonist at US28 in a physiologically relevant model system.

US28 is an carly gene, being transcribed as early as 2 hours after HCMV infection of permissive cells, such as fibroblasts. Consequently, cells permissive to HCMV infection express a receptor that functions in the absence of any ligand and may influence the cellular machinery early after infection. Notably, US28 constitutively activates pathways such as PLC and NF-κB, which are important for viral replication. The identification of the small inverse agonist the compound of Formula X provides a tool to investigate the role of US28 in activating a cell to allow or enhance viral replication. Studies conducted with a US28 deletion mutant virus have shown that, although US28 is not required for viral growth in culture, its removal results in a four to fivefold reduction in virus peak titers (see in this respect the cited article of Vieira *et al.*).

US28 is transcribed not only in cells permissive to CMV infection, but also in latently infected cells, such as monocytes, suggesting that US28 may affect a wide range of cell types. In monocytes, the transcription factor NF-κB promotes expression of over 100 target genes, mostly involved in the regulation of the immune response.

Without wishing to be bound by any theory, it is believed that US28, through activation of PLC and NF-κB pathways, might alter expression of such proteins, resulting in alteration of the immune response in favour of viral survival and spreading.

Since HCMV infection is a co-factor in HIV disease progression, the compounds of the present invention can suitably be used in the inhibition of AIDS. In e.g. HIV-positive infants CMV infection increases chances of progression to AIDS, impaired brain growth and motor deficits. Cellular entry of the HIV-1 virus is mediated via interaction of the viral glycoprotein 120 (gp 120) with CD4 and a second co-receptor, which belongs to the chemokine receptor family. The best characterized co-receptors are CCR5, which mediates entry of monotropic (R5) HIV strains, and CXCR4, which mediates entry of lymphotropic (X4) HIV strains. The HCMV encoded US28 can also enhance viral entry for both R5- and X4-tropic HIV strains *in vitro* (*cfr.* Pleskoff *et al,* Science 276 (1997), 1874-1878), giving a molecular basis to the epidemiological link between HCMV and HIV-1 infection. The results with 293T cells confirm US28 as a potential co-receptor for CCR5-tropic HIV strains. Particularly, the inverse agonist the compound of Formula X at a concentration of 1 µM inhibits 60 % of viral entry, suggesting that small ligands acting at US28 might have anti-HIV properties.

The small non-peptidergic compounds of Formula I are inverse agonists at the HCMV-encoded chemokine receptor US28. These compounds, and particularly the compound of Formula X, inhibit US28-mediated HIV-1 infection.

The identification of an inverse agonist provides a tool for further dissecting the role of US28 and its constitutive activity in HCMV infection. In addition, it might serve as a potential lead for innovative antiviral drug design.

## Claims

1. Non-peptidergic compound being an inverse agonist acting on HCMV encoded US28 receptor.

2. Compound according to claim 1, having a structure of formula (I), wherein:
A is selected from the group consisting of C(X)(Ar), N-(CH₂)ₖ-Y-Ar, O, NH and N-R₄;
X is H, OH, OR₄, or R₄;
R₄ is, independently, a C₁₋₃ alkyl group, optionally substituted by inert substituents, such as one or more halogen atoms;
Ar is 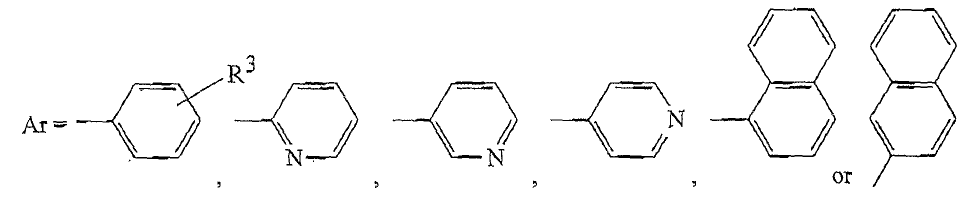
k is an integer of 0-6;
Y is C(OH)(H) or C=O;
B is CH or N;
with the proviso that when A is NH, B is not N;
m is an integer of 1-3;
n is an integer of 0-4;
R₅ is H, OH, CN, R₄, or CH₂NH₂;
R₁, R₂ and R₃, independently, represent H, OH, OR₄, a halogen atom, CN, a straight or branched C₁₋₅ alkyl that can optionally be substituted with one or more halogen atoms; and
P and Q represent each a hydrogen atom, or P and Q together are a -SCH₂-, a -CH₂S-, a -OCH₂-, a -CH₂O-, a -CH=CH- group or a C₁₋₂ alkylene group.

3. The compound of claim 2, wherein is either a halogen atom and preferably a chlorine atom, or a hydroxyl group.

4. The compound of any one of the preceding claims having formula X:

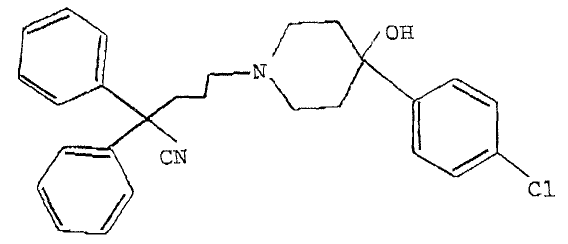

5. Use of any one of the compounds as defined in any one of the preceding claims in the manufacture of a medicament having inverse agonist activity on HCMV encoded US28 receptor.

6. Use of an inverse agonist acting at US28 in the manufacture of a medicament for inhibiting or the prevention of HIV entry into cells.

7. The use of claim 5, wherein the medicament is to treat or to prevent HCMV infection.

8. The use of claim 5, 6, or 7 wherein the medicament is a prophylacticum for an individual having a compromised immune system, or for an individual expected to encounter a suppressed immune system.

9. Use of any one of the compounds as defined in any one of the claims 1-4 in the manufacture of a medicament for inhibiting viral infections.

10. The use of claim 5, 6 or 9, wherein the medicament is to treat an HIV infection, such as in AIDS.

11. Use of a compound according to any one of claims 1-4 in the manufacture of a diagnosticum for detecting or imaging CMV infection in a host, which diagnosticum is intended to be administered to a host suspected to have a CMV infection or to a sample taken from said host, after which binding of said compound in the host or sample is measured.

12. An assay using constitutively active virus-encoded G Protein-Coupled Receptors as screening method of non-peptidergic ligands for these receptors.

13. The assay of claim 12, using US28-mediated constitutive inositol phosphate production.
